# EUROPÄISCHE PATENTSCHRIFT

(11) **EP 0 632 799 B1**
(45) Veröffentlichungstag und Bekanntmachung des Hinweises auf die Patenterteilung: **09.10.1996**
(21) Anmeldenummer: 93906524.9
(22) Anmeldetag: 13.03.1993
(51) Int. Cl.: C07C 201/12, C07C 205/56, C07D 303/40

(54) **VERFAHREN ZUR HERSTELLUNG VON CHLORSUBSTITUIERTEN OLEFINISCHEN VERBINDUNGEN**
PROCESS FOR PRODUCING CHLORINE-SUBSTITUTED OLEFINE COMPOUNDS
PROCEDE DE FABRICATION DE COMPOSES OLEFINIQUES CHLOROSUBSTITUES

(30) Priorität: 24.03.1992 DE 4209497
(43) Veröffentlichungstag der Anmeldung: 11.01.1995
(73) Patentinhaber: BASF AKTIENGESELLSCHAFT, 67056 Ludwigshafen (DE)
(72) Erfinder: SCHAEFER, Bernd, D-6749 Dierbach (DE); TROETSCH-SCHALLER, Irene, D-6710 Frankenthal (DE)
(86) Internationale Anmeldenummer: EP9300586
(87) Internationale Veröffentlichungsnummer: WO9319034

(56) Entgegenhaltungen:
- CH-A- 605 882
- DE-B- 1 096 899
- FR-A- 2 662 161

## Beschreibung

Die vorliegende Erfindung betrifft ein neues Verfahren zur Herstellung von chlorsubstituierten olefinischen Verbindungen der allgemeinen Formel I 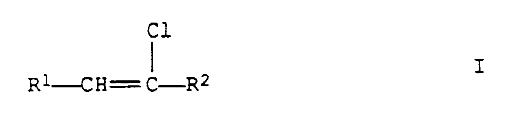 in der R¹ einen C-organischen Rest und R² eine der folgenden Gruppen bedeuten: -CN, -CO-R³, -CO-S-R³, -CO-O-R³ oder -CO-N(R⁴,R⁵), wobei R³ für einen C-organischen Rest und R⁴ und R⁵ für Wasserstoff oder einen C-organischen Rest stehen.

Zur Herstellung von α-Chlor-zimtsäureestern als Verbindungen I (R¹ = Phenyl; R² = -COOR³) sind mehrere Methoden bekannt, die jedoch in verschiedener Hinsicht zu wünschen übrig lassen. So kann man an die Doppelbindung der entsprechenden Zimtsäureester zunächst Chlor addieren und aus den α,β-dichlorierten 3-Phenylpropionsäureestern wieder Chlorwasserstoff abspalten (vgl. z.B. J. March, Advanced Organic Chemistry, Mc Graw-Hill International Book Company, Auckland, 2nd. Ed., 1977, Seite 739ff). Nachteilig ist hierbei, daß die Reaktion wenig selektiv verläuft.

Eine weitere Methode besteht in der Verknüpfung von Benzaldehyd (oder einem substituierten Benzaldehyd) mit
a) einem Phosphoniumsalz des Bausteins -CH(Cl)-R² nach Wittig {G. Wittig, G. Geisler, Liebigs Ann. Chem. 44, 580 (1953) sowie G. Märkl, Chem. Ber. 94, 2996 (1961)} oder
b) einem entsprechenden monochlorierten Phosphonoessigsäureester nach Horner {Horner, Hoffmann, Wippel, Chem. Ber. 91, 61 (1958); Horner, Hoffmann, Wippel, Klahre, Chem. Ber. 92, 2499 (1959) sowie McKenna, Khawli, J. Org. Chem. 51 , 5467 (1986)}.

Diese Arbeitsweise ist jedoch nicht zuletzt im Hinblick auf verfahrestechnische Schwierigkeiten nachteilig.

Weiterhin ist bekannt, daß die Einwirkung von Phosgen, Phosphoroxychlorid, Phosphortri- oder Phosphorpentachlorid auf Oxirane der Formel II' 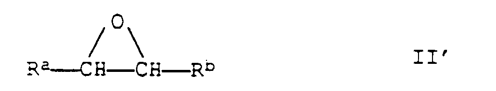 wobei R^{a} und R^{b} Wasserstoff, Alkyl oder Phenyl oder zusammen einen carbocyclischen Ring bedeuten, u.a. in überschüssigem Dimethylformamid, zu 1,2-Dichlorverbindungen führt {W. Ziegenbein, K.-H. Hornung, Chem. Ber. 95, 2976 (1962)}.

Gemäß der Lehre der DE-A 10 96 899 erhält man durch Umsetzung von aliphatischen oder cycloaliphatischen 1,2-Epoxiden, die Aryl- oder Aralkylsubstituenten tragen können, mit Additionsverbindungen aus Phosphoroxychlorid oder Phosgen und einem N,N-Dialkylamid und anschließender Hydrolyse 1-Acyloxy-2-chloralkyl-Derivate von Alkanen oder Cycloalkanen.

Der Erfindung lag die Aufgabe zugrunde, ausgehend von billigen und technich leicht handhabbaren Verbindungen eine einfache und technisch wirtschaftliche Methode zur Herstellung der chlorsubstituierten olefinischen Verbindungen I bereitzustellen.

Demgemäß wurde ein Verfahren zur Herstellung von chlorsubstituierten olefinischen Verbindungen der Formel I gefunden, welches dadurch gekennzeichnet ist, daß man ein Oxiran der allgemeinen Formel II, 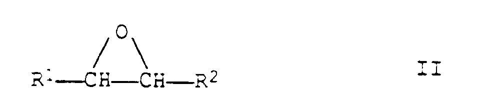 in Gegenwart eines Carbonsäureamids (IIIa) oder eines Lactams (IIIb) in flüssiger Phase mit einem Chlorierungsmittel (IV) umsetzt.

Außerdem wurden neue Oxirane der allgemeinen Formel IIa 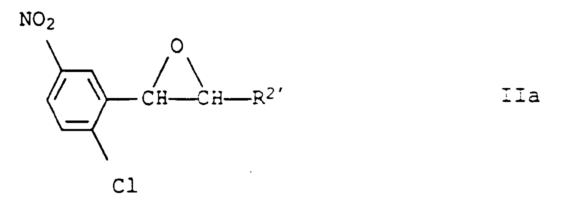 wobei R^{2'} Cyano, C₁-C₆-Alkylcarbonyl, C₁-C₆-Alkoxycarbonyl oder C₁-C₆-Alkylthiocarbonyl bedeuten, gefunden.

Die als Ausgangsprodukte dienenden Oxirane der Formel II sind nach an sich bekannten Methoden {z. B. M.S. Newman, B. J. Magerlein, Org. React. 5, 413 (1949)} herstellbar.

Die neuen Oxirane IIa erhält man vorzugsweise durch Umsetzung von 2-Chlor-5-nitrobenzaldehyd mit einem Acrylnitril-, Methylalkylketon- oder Essigester-Derivat der Formel L-CH₂-R^{2'}, wobei L eine nucleophile Abgangsgruppe, insbesondere Chlor, bedeutet.

Die Umsetzung erfolgt in einem inerten Lösungs- oder Verdünnungsmittel in Gegenwart einer starken Base, beispielsweise einem Alkalimetallalkoholat wie Natriummethylat.

Als Lösungs- oder Verdünnungmittel eignen sich insbesondere niedere Alkohole wie Methanol, Ethanol und Isopropanol. Zweckmäßigerweise arbeitet man in dem Alkohol, dessen Alkoholat Bestandteil der Base ist.

Die Reaktionstemperatur liegt im allgemeinen zwischen 0 und 40°C.

In der Regel arbeitet man unter Atmosphärendruck oder unter dem Eigendruck des jeweiligen Lösungsmittels.

Die erfindungsgemäße Chlorierung der Oxirane II erfolgt in Gegenwart eines Carbonsäureamids oder eines Lactams, wobei sich beispielsweise Verbindungen der Formel IIIa 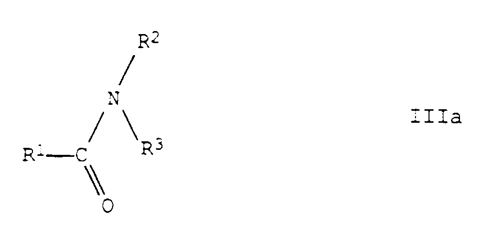 in der die Substituenten die folgende Bedeutung haben:
R¹ = Wasserstoff, C₁-C₆-Alkyl oder Phenyl und
R², R³ = C₁-C₆-Alkyl oder Phenyl oder
R² und R³ zusammen mit dem gemeinsamen Stickstoffatom Pyrrolidinyl, Piperidinyl oder Morpholinyl; und Verbindungen der Formel IIIb 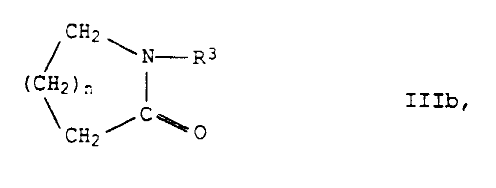 wobei n 0, 1 oder 2 und R³ C₁-C₆-Alkyl oder Phenyl bedeuten,
als besonders geeignet erwiesen haben.

Nach bisheriger Erkenntnis sind N,N-Dimethylformamid, N-Formylmorpholin, N-Formylpiperidin, N-Methyl-N-phenylformamid und N-Methylpyrrolidon besonders vorteilhaft; ganz besonders bevorzugt ist Dimethylformamid.

Als Chlorierungsmittel kommen vornehmlich Vilsmeier-Salze oder nicht-oxidierende Chlorierungsmittel in Betracht, also beispielsweise Sulfurylchlorid, Thionylchlorid, Acetylchlorid, Benzoylchlorid, Pivalinsäurechlorid, Bis-(trichlormethyl)-carbonat, Oxalyldichlorid, Phosphoroxychlorid, Phosphortrichlorid, Phosphorpentachlorid, Methansulfonsäurechlorid, Chlorsulfonsäure, Phosgen oder Chlorameisensäuretrichlormethylester (vgl. auch H. Eilingsfeld, M. Seefelder, H. Weidinger, Chem. Ber. 96 (1963) 2691; C. Jutz, Advances in Org. Chem. 9 (1976) 225; M. Grdinic, V. Hahn, J. Org. Chem. 30 (1965) 2381; H. Eilingsfeld, M. Seefelder, H. Weidinger, Angew. Chem. 72 (1960) 836).

Eine vorteilhafte Ausführungsform des Verfahrens besteht darin, aus dem verwendeten Carbonsäureamid (IIIa) oder Lactam (IIIb) zuerst mit einem geeigneten nicht-oxidierenden Chlorierungsmittel, insbesondere mit Thionylchlorid, Acetylchlorid, Benzoylchlorid, Oxalyldichlorid, Phosphoroxychlorid, Phosphortrichlorid, Phosphorpentachlorid, Chlorsulfonsäure, Phosgen oder Chlorameisensäuretrichlormethylester, das entsprechende Vilsmeier-Salz (IVa oder IVb) herzustellen und die herbei erhaltene Lösung, die auch noch überschüssiges Carbonsäureamid (IIIa) oder Lactam (IIIb) enthält, mit dem Oxiran II umzusetzen: 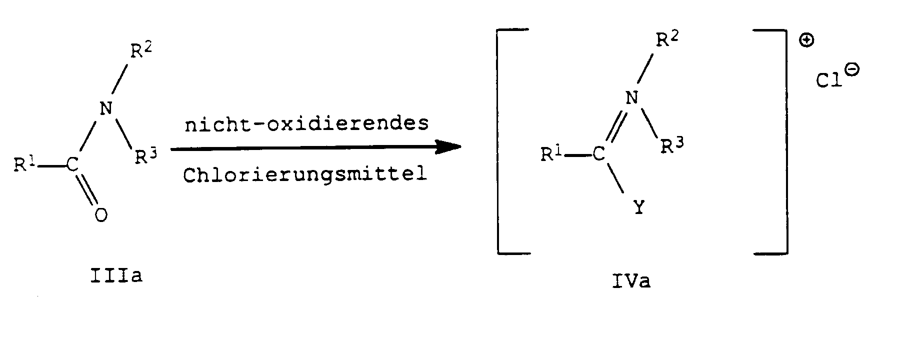 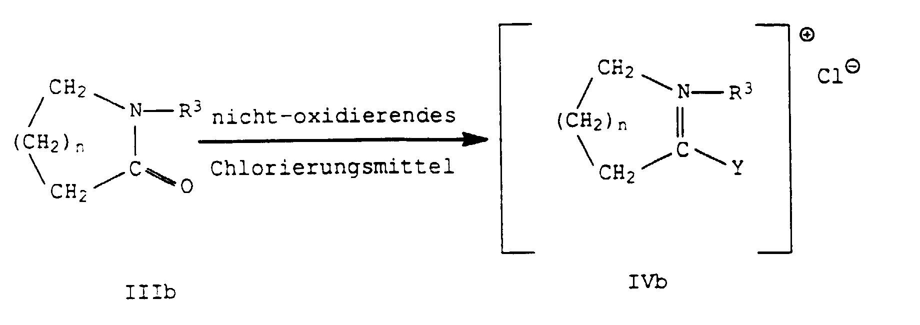 Y ist abhängig vom eingesetzten Chlorierungsmittel und steht insbesondere für Chlor, -OSOCl, -O-CO-CH₃, -O-CO-phenyl, -OPO(Cl)₂, -OP(Cl)₂ oder -OP(Cl)₄;
Da die Reihenfolge der Zugabe der Reaktionspartner normalerweise keinen Einfluß auf die Produktbildung hat, ist es unerheblich, ob das Oxiran II bereits vor oder erst nach Zugabe des Chlorierungsmittel mit dem Carbonsäureamid oder Lactam vermischt wird.

Andererseits kann das Vilsmeier-Salz aber auch nach der Herstellung isoliert und gereinigt werden und erst dann mit dem Oxiran II zur Reaktion gebracht werden, wobei man auch in einem anderen Carbonsäureamid oder Lactam als dem für die Herstellung des Vilsmeier-Salzes verwendeten, arbeiten kann.

Bei ungenügender thermischer Stabilität der Vilsmeier-Salze kann es hierbei vorteilhaft sein, erst das Carbonsäureamid (IIIa) oder Lactam (IIIb) mit Chlorwasserstoff zu sättigen und danach das Vilsmeier-Salz (IVa oder IVb) und das Oxiran II einzubringen.

Zur Verbesserung der Löslichkeit der Reaktionspartner kann außerdem ein inertes Lösungs- oder Verdünnungsmittels zugesetzt werden, das sich unter den Chlorierungsbedingungen inert verhält.

Als inerte Lösungsmittel kommen hierfür Petrolether, aromatische Kohlenwasserstoffe wie Toluol und o-, m-, p-Xylol, chlorierte Kohlenwasserstoffe wie Dichlormethan, Trichlormetan, Tetrachlormethan, 1,1,1-Trichlorethan und 1,2-Dichlorethan oder aromatische Halogenkohlenwasserstoffe wie Chlorbenzol in Betracht.

Vorzugsweise arbeitet man jedoch ohne zusätzliches Lösungsmittel in einem Carbonsäureamid (IIIa) oder Lactam (IIIb).

Die Menge an Carbonsäureamid oder Lactam und gegebenenfalls inertem Lösungsmittel ist so zu bemessen, daß mindestens ein Teil der Reaktionspartner in Lösung geht.

Vorteilhaft verwendet man in etwa stöchiometrische Mengen an Chlorierungsmittel und Oxiran II. Zur Vermeidung von Nebenprodukten kann es jedoch vorteilhaft sein, die Reaktion bei einem geringeren Umsatz zu beenden. In diesem Fall verwendet man unterstöchiometrische Mengen an Chlorierungsmittel, bis etwa 10 mol-%.

Die optimale Reaktionstemperatur ist abhängig von den jeweiligen Reaktionspartnern. Sie liegt im allgemeinen etwa zwischen 0 und 150°C, bevorzugt zwischen 20 und 100°C.

Die Reaktion ist vom Druck nicht erkennbar abhängig, so daß man vorteilhaft bei atmosphärischem Druck arbeitet. Im Falle leichtflüchtiger Reaktionspartner (z.B. Phosgen) kann jedoch auch ein höherer Druck bis etwa 20 bar, vorzugsweise bis 6 bar, empfehlenswert sein.

Zweckmäßigerweise hält man die Reaktionsbedingungen aufrecht, bis kein Oxiran II im Reaktionsgemisch mehr nachgewiesen werden kann (z.B. mittels Dünnschichtchromatographie, Hochdruckflüssigkeitschromatographie und Gaschromatographie). Die Aufarbeitung auf das Verfahrensprodukt hin erfolgt dann in der Regel nach herkömmlichen Verfahren wie Destillation, Filtration, Zentrifugation oder durch Zugabe von Wasser und anschließender Extraktion.

Das erfindungsgemäße Verfahren ist diskontinuierlich, z.B. in einem Rührreaktor, auszuführen. Die einfache Durchführbarkeit bietet jedoch den Vorteil, daß man es auf kontinuierliche Arbeitsweise, beispielsweise unter Verwendung eines Reaktionsrohrs oder einer Rührreaktorkaskade, umstellen kann.

Die erhaltenen Rohprodukte können gewünschtenfalls weiter gereinigt werden, z.B. durch Kristallisation, Rektifikation cder mittels chromatographischer Methoden.

Die Verfahrensprodukte I fallen im allgemeinen als Mischung aus cis- und trans-Isomeren (bezogen auf die olefinische Doppelbindung) an. Die erfindungsgemäße Chlorierung eines optisch aktiven Oxirans II, das eines der beiden Isomeren im Überschuß enthält, führt zu cis-/trans-Isomerengemischen I, bei denen ebenfalls eine Konfiguration überwiegt.

Im Hinblick auf die gewünschten Verfahrensprodukte I sind die folgenden Substituenten R¹ und R² von besonderer Bedeutung:
- R¹: ein iso- oder heterocyclischer aromatischer Rest, insbesondere Phenyl- oder Pyridyl, die beide unsubstituiert sein oder ein bis drei Substituenten tragen können, ausgewählt aus einer Gruppe bestehend aus Fluor, Chlor, Brom, Nitro oder einem C-organischen Rest mit 1 bis 12 C-Atomen, der gewünschtenfalls
a) ein Sauerstoff- oder Schwefelatom enthalten und/oder
b) partiell oder vollständig halogeniert sein kann;
- R²: -CN oder -CO-R³, wobei R³ einen C-organischen Rest mit 1 bis 12 C-Atomen bedeutet.

Unter den C-organischen Resten mit 1 bis 12 C-Atomen sind beispielsweise die folgenden Gruppen zu verstehen:
- eine verzweigte oder unverzweigte C₁-C₆-Alkylgruppe, die noch ein oder zwei C₁-C₄-Alkoxyreste, vorzugsweise Methoxy und Ethoxy und/oder C₁-C₄-Alkylthioreste, vorzugsweise Methylthio, tragen kann;
- eine Cyclopropyl-, Cyclobutyl-, Cyclopentyl- oder Cyclohexylgruppe;
- eine C₃-C₆-Alkenylgruppe, vorzugsweise 2-Propenyl und 2-Butenyl;
- eine C₃-C₆-Alkinylgruppe, vorzugsweise 2-Propinyl und 2-Butinyl;
- eine C₁-C₄-Alkoxygruppe, vorzugsweise Methoxy und Ethoxy;
- eine C₁-C₄-Alkylthiogruppe;
- eine Arylgruppe, insbesondere die Phenylgruppe, eine C₁-C₄-Alkylphenylgruppe wie o-, m-, p-Tolyl, eine C₁-C₄-Alkoxyphenylgruppe wie o-, m-, p-Methoxyphenyl, eine Halogenphenylgruppe wie o-, m-, p-Fluorphenyl, o-, m-, p-Chlorphenyl und o-, m-, p-Bromphenyl, sowie die o-, m-, p-Nitrophenyl-, o-, m-, p-(Trifluormethyl)-phenyl- oder die o-, m-, p-Biphenylgruppe.

Im Hinblick auf die Folgeprodukte, meist α-chlorierte Zimtsäureester der Formel V 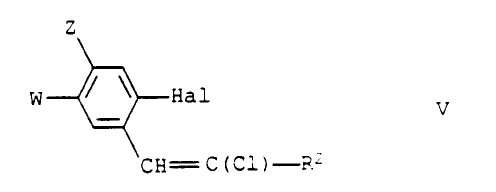 in der Hal für Fluor, Chlor oder Brom,
Z für Wasserstoff, Fluor, Chlor oder Brom und
W für einen der folgenden heterocyclischen Reste stehen: 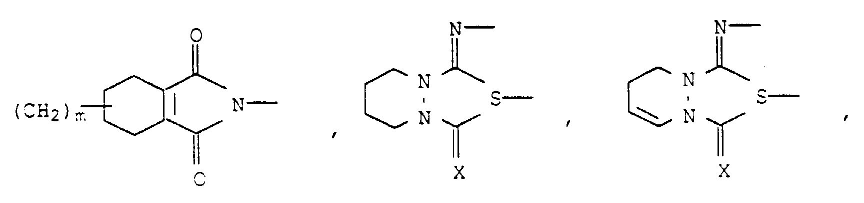 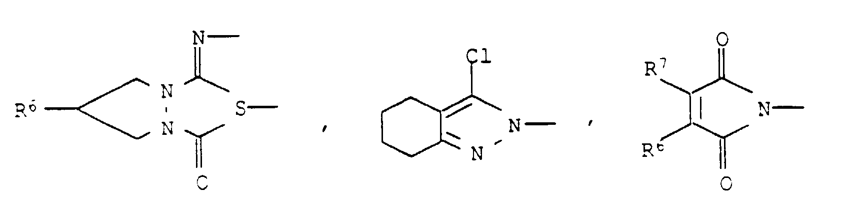 (m = 0 oder 1; X = Sauerstoff oder Schwefel: R⁶, R⁷ = Wasserstoff oder C₁-C₄-Alkyl)
   bedeutet R¹ besonders bevorzugt eine mono- oder dichlorierte meta-Anilinogruppe und R² eine Cyano-, C₁-C₆-Alkylcarbonyl- oder C₁-C₆-Alkoxycarbonylgruppe.

Die in der Definition der Substituenten verwendeten Sammelbegriffe
- Halogen,
- C₁-C₆-Alkyl, C₁-C₄-Alkyl, C₁-C₂-Alkoxy, C₁-C₄-Alkylthio,
- C₃-C₆-Alkenyl, C₃-C₆-Alkinyl, C₃-C₆-Alkinyloxy,
- C₁-C₆-Alkylcarbonyl, C₁-C₆-Alkoxycarbonyl
stellen Kurzschreibweisen für eine individuelle Aufzählung der einzelnen Gruppenmitglieder dar. Sämtliche Alkyl-, Alkoxy-, Alkylthio-, Alkenyl- und Alkinylteile können geradkettig oder verzweigt sein.

Im einzelnen bedeuten beispielsweise
- C₁-C₄-Alkyl: Methyl, Ethyl, n-Propyl, 1-Methylethyl, n-Butyl, 1-Methylpropyl, 2-Methylpropyl und 1,1-Dimethylethyl:
- C₁-C₆-Alkyl: C₁-C₄-Alkyl sowie n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 1,2-Dimethylbutyl, 2,3-Dimethylbutyl, 3,3-Dimethylbutyl, 1-Ethyl-butyl, 2-Ethylbutyl, 1,1,2-Trimethylpropyl, 1,2,2-Trimethyl-propyl, 1-Ethyl-1-methylpropyl und 1-Ethyl-2-methylpropyl;
- C₁-C₄-Alkoxy: Methoxy, Ethoxy, n-Propoxy, 1-Methyl-ethoxy, n-Butoxy, 1-Methyl-propoxy, 2-Methyl-propoxy und 1,1-Dimethylethoxy;
- C₁-C₄-Alkylthio: Methylthio, Ethylthio, n-Propylthio, 1-Methylethylthio, n-Butylthio, 1-Methylpropylthio, 2-Methylpropylthio und 1,1-Dimethylethylthio;
- C₃-C₆-Alkenyl: 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 3,3-Dimethyl-2-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl:
- C₃-C₆-Alkinyl: 1-Propinyl, 2-Propinyl, 1-Butinyl, 2-Butinyl, 3-Butinyl, 1-Pentinyl, 2-Pentinyl, 3-Pentinyl, 4-Pentinyl, 3-Methyl-1-butinyl, 4-Methyl-1-butinyl, 1-Methyl-2-butinyl, 4-Methyl-2-butinyl, 1-Methyl-3-butinyl, 2-Methyl-3-butinyl, 1-Hexinyl, 2-Hexinyl, 3-Hexinyl, 4-Hexinyl, 5-Hexinyl, 3-Methyl-1-pentinyl, 4-Methyl-1-pentinyl, 5-Methyl-1-pentinyl, 1-Methyl-2-pentinyl, 4-Methyl-2-pentinyl, 5-Methyl-2-pentinyl, 1-Methyl-3-pentinyl, 2-Methyl-3-pentinyl, 5-Methyl-3-pentinyl, 1-Methyl-4-pentinyl, 2-Methyl-4-pentinyl und 3-Methyl-4-pentinyl;
- C₁-C₆-Alkylcarbonyl: Methylcarbonyl, Ethylcarbonyl, n-Propylcarbonyl, 1-Methylethyl-carbonyl, n-Butylcarbonyl, 1-Methylpropylcarbonyl, 2-Methylpropylcarbonyl, 1,1-Dimethylethylcarbonyl, n-Pentylcarbonyl, 1-Methylbutylcarbonyl, 2-Methylbutylcarbonyl, 3-Methylbutylcarbonyl, 1,1-Dimethylpropylcarbonyl, 1,2-Dimethylpropylcarbonyl, 2,2-Dimethylpropylcarbonyl, 1-Ethylpropylcarbonyl, n-Hexylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl und 1-Ethyl-2-methylpropylcarbonyl;
- C₁-C₆-Alkoxycarbonyl: Methoxycarbonyl, Ethoxycarbonyl, n-Propyloxycarbonyl, 1-Methyl-ethoxycarbonyl, n-Butoxycarbonyl, 1-Methylpropyloxycarbonyl, 2-Methylpropyloxycarbonyl, 1,1-Dimethylethoxycarbonyl, n-Pentoxycarbonyl, 1-Methylbutyloxycarbonyl, 2-Methylbutyloxycarbonyl, 3-Methylbutyloxycarbonyl, 2,2-Dimethylpropyloxycarbonyl, 1-Ethylpropyloxycarbonyl, n-Hexoxycarbonyl, 1,1-Dimethylpropoxycarbonyl, 1,2-Dimethylpropyloxycarbonyl, 1-Methylpentyloxycarbonyl, 2-Methylpentyloxycarbonyl, 3-Methylpentyloxycarbonyl, 4-Methylpentyloxycarbonyl, 1,1-Dimethylbutyloxycarbonyl, 1,2-Dimethylbutyloxycarbonyl, 1,3-Dimethylbutyloxycarbonyl, 2,2-Dimethylbutyloxycarbonyl, 2,3-Dimethylbutyloxycarbonyl, 3,3-Dimethylbutyloxycarbonyl, 1-Ethylbutyloxycarbonyl, 2-Ethylbutyloxycarbonyl, 1,1,2-Trimethylpropyloxycarbonyl, 1,2,2-Trimethylpropyloxycarbonyl, 1-Ethyl-1-methylpropyloxycarbonyl und 1-Ethyl-2-methylpropyloxycarbonyl.

Die nach dem erfindungsgemäßen Verfahren auf einfache Weise herzustellenden chlorsubstituierten olefinischen Verbindungen I sind wertvolle Zwischenprodukte zur Synthese von Farbstoffen, Arzneimitteln und Pflanzenschutzmitteln, insbesondere von Herbiziden und Wachstumsregulatoren, wie sie beispielsweise in den folgenden Druckschriften beschrieben sind: EP-A 240 659, EP-A 379 911, DE-A 40 42 194.

### Beispiel 1

### 3-(2-Chlor-5-nitrophenyl)-glycidsäureethylester [Ausgangsprodukt II' (R¹ = 2-Chlor-5-nitrophenyl; R² = Ethoxycarbonyl)]

Zu einer Mischung aus 60 l Ethanol und 56,7 kg einer 21 gew.-%igen Lösung von Natriumethylat in Ethanol (= 175 mol NaOC₂H₅) wurden bei 20-25°C innerhalb von 2 Stunden 27,8 kg (150 mol) 2-Chlor-5-nitrobenzaldehyd und anschließend innerhalb von 1 Stunde 20,4 kg (166,6 mol) Chloressigsäureethylester gegeben. Nach anschließendem ca. 15 stündigem Rühren trennte man den Feststoffanteil möglichst vollständig ab und trocknete ihn dann unter reduziertem Druck (100 mbar) bei 40°C. Ausbeute: 87%; Fp.: 79°C.

### Beispiel 2

### α,2-Dichlor-5-nitrozimtsäureethylester [I: R¹ = 2-Chlor-5-nitrophenyl; R² = Ethoxycarbonyl]

11,7 g einer 36,89 gew.-%igen Lösung von Chlormethylendimethyl-iminiumchlorid-Hydrochlorid (26,2 mmol) in Dimethylformamid-Hydrochlorid wurden in 15 ml Dimethylformamid gelöst. Zu der erhaltenen Lösung tropfte man bei 25 - 30°C eine Lösung von 6,8 g (25 mmol) 3-(2-Chlor-5-nitrophenyl)-glycidsäureethylester in 15 ml Dimethylformamid. Anschließend erhitzte man 4 Stunden auf 80°C, wonach das Lösungsmittel unter reduziertem Druck abdestilliert wurde. Der Rückstand wurde mit 50 ml Wasser gewaschen und dann aus 40 ml Ethanol umkristallisiert. Ausbeute: 72% (E/Z-Isomerenverhältnis = 3,4 : 88,5); Fp.: 91°C.

### Beispiel 3

### α,2-Dichlor-5-nitrozimtsäureethylester [I; R¹ = 2-Chlor-5-nitrophenyl: R² = Ethoxycarbonyl]

Zu einer Mischung aus 27,5 g (0,1 mol)
3-(2-Chlor-5-nitro-phenyl)-glycidsäureethylester und 200 ml Dimethylformamid wurden bei 50°C innerhalb einer halben Stunde 15 g (0,15 mol) Phosgen eingegast. Anschließend erhitzte man 5,5 Stunden auf 80°C und ließ dann die Mischung auf ca. 25°C abkühlen. Nach Entfernen des Lösungsmittels unter reduziertem Druck wurde der Rückstand aus 200 ml Ethanol umkristallisiert. Danach wurde das Rohprodukt mit Wasser gewaschen und unter reduziertem Druck bei 40 °C getrocknet. Ausbeute: 6 % (GC: 98,8 Flächenprozent; E/Z-Isomerenverhältnis = 8,9 : 80,9): Fp.: 91°C.

### Beispiel 4

### α,2-Dichlor-5-nitrozimtsäureethylester [I; R¹ = 2-Chlor-5-nitrophenyl: R² = Ethoxycarbonyl]

Zu einer auf 100 °C erwärmten Lösung von 13,6 g (50 mmol) 3-(2-Chlor-5-nitrophenyl)-glycidsäureethylester in 100 ml Dimethylformamid wurden innerhalb von 20 Minuten 7,1 g (60 mmol) Thionylchlorid getropft. Nach 4 Stunden Rühren bei 100°C entfernte man das Lösungsmittel unter reduziertem Druck. Das Rohprodukt wurde noch warm mit 40 ml Ethanol und 10 ml Wasser versetzt, wonach man kurz umrührte, dann auf 0°C kühlte und den Feststoffanteil abtrennte. Das Rohprodukt wurde zweimal mit einem Gemisch aus je 20 ml Ethanol und 20 ml Wasser gewaschen und anschließend bei 50°C und 100 mbar getrocknet. Ausbeute: 73% (GC: 92,1/5,9 Flächenprozent; E/Z-Isomerenverhältnis = 6,5 : 82,4); Fp.: 90°C.

### Beispiel 5

### α,2-Dichlor-5-nitrozimtsäureethylester [I; R¹ = 2-Chlor-5-nitrophenyl: R² = Ethoxycarbonyl]

Zu einer Lösung von 13,6 g (50 mmol) 3-(2-Chlor-5-nitrophenyl)glycidsäureethylester in 100 ml Dimethylformamid wurden innerhalb von 15 Minuten 7 g (55 mmol) Oxalylchlorid getropft. Man rührte die Mischung noch 1 Stunde bei 20-25°C und erwärmte anschließend 10 Stunden auf 80°C. Danach wurde das Lösungsmittel unter reduziertem Druck entfernt. Der Rückstand wurde in 30 ml Ethanol gerührt. Nach Kühlung auf 0°C trennte man den Feststoffanteil ab, wusch ihn mit wenig kaltem Ethanol nach und trocknete ihn bei 50°C und 100 mbar. Ausbeute: 52% (GC: 97,9 Flächenprozent; E/Z-Isomerenverhältnis = 10,0 : 74,0); Fp.: 94°C.

### Beispiel 6

### α,2-Dichlor-5-nitrozimtsäureethylester [I; R¹ = 2-Chlor-5-nitrophenyl: R² = Ethoxycarbonyl]

Beipiel 4 wurde mit 8,4 g (55 mmol) Phosphorylchlorid als Chlorierungsmittel wiederholt. Die Reaktionsmischung wurde 5 Stunden auf 100°C erhitzt, wonach man das Lösungsmittel unter reduziertem Druck entfernte. Der Rückstand wurde mit 40 ml Ethanol verrührt. Dann kühlte man auf 0°C, trennte den Feststoffanteil ab, wusch ihn noch zweimal mit je 40 ml Wasser und trocknete ihn schließlich unter reduziertem Druck (100 mbar) bei 50°C. Ausbeute: 63% (GC: 95,8 Flächenprozent: E/Z-Isomerenverhältnis = 6,4 : 82,8); Fp.: 92°C.

### Beispiel 7

### α-Chlor-3-nitrozimtsäureethylester [I; R¹ = 3-Nitrophenyl; R² = Ethoxycarbonyl]

Zu einer Mischung aus 14,1 g (0,11 mol) Chlormethylendimethyliminiumchlorid und 150 ml Dimethylformamid wurde innerhalb von 15 Minuten eine Lösung von 23,7 g (0,1 mol) 3-(3-nitrophenyl)glycidsäureethylester in 50 ml Dimethylformamid getropft. Die Mischung wurde 1 Stunde bei 20-25°C und dann noch 2,5 Stunden bei 80°C gerührt. Danach entfernte man das Lösungsmittel unter reduziertem Druck. Der Rückstand wurde mit 50 ml Ethanol verrührt. Nach Kühlung auf (-10)°C trennte man den Feststoffanteil ab, wusch ihn mit wenig kaltem Ethanol nach und trocknete ihn bei 50°C und 100 mbar. Ausbeute: 34% (GC: 99,6 Flächenprozent; isomerenrein Z); Fp.: 75°C.

### Beispiel 8

### α,2-Dichlor-5-nitrozimtsäureethylester [I; R¹ = 2-Chlor-5-nitrophenyl; R² = Ethoxycarbonyl]

Zu einer Mischung aus 66 g (0,225 mol) 3-(2-Chlor-5-nitrophenyl)glycidsäureethylester (Reinheit 92,6 %), 11,93 g (0,113 mol) Natriumcarbonat und 131,2 g (1,797 mol) Dimethylformamid wurden innerhalb von 25 min bei 100°C 31,79 g (0,405 mol) Acetylchlorid getropft. Nach 6 Stunden Rühren bei 20 bis 25°C trennte man den Feststoffgehalt ab, wonach die erhaltene Lösung bei maximal 64°C unter 10 mbar Druck eingeengt wurde. Nach waschen des Rückstandes mit je 30 ml Ethanol, Wasser und Petrolether und trocknen bei 50°C unter reduziertem Druck erhielt man einen hellbraunen Feststoff. Ausbeute: 68 % (GC: 95,2 Flächenprozent).
200MHz-¹H-NMR (in CDCl₃; TMS als interner Standard):
- δ [ppm] =: 1.42 (t,3H,CH₃); 4.42 (q,2H,CH₂);
- 7.62 (d,1H,Aromat); 8.10 (s,1H,CH);
- 8.21 (dd,1H,Aromat); 8.82 (d,1H,Aromat).

## Patentansprüche

1. Verfahren zur Herstellung von chlorsubstituierten olefinischen Verbindungen der allgemeinen Formel I 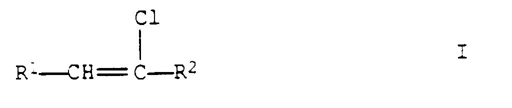 in der R¹ einen C-organischen Rest und R² eine der folgenden Gruppen bedeuten: -CN, -CO-R³, -CO-S-R³, -CO-O-R³ oder -CO-N(R⁴,R⁵),
wobei R³ für einen C-organischen Rest und R⁴ und R⁵ für Wasserstoff oder einen C-organischen Rest stehen,
dadurch gekennzeichnet, daß man ein Oxiran der allgemeinen Formel II, 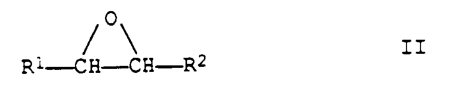 in Gegenwart eines Carbonsäureamids (IIIa) oder eines Lactams (IIIb) in flüssiger Phase mit einem Chlorierungsmittel (IV) umsetzt.

2. Verfahren nach Anspruch 1, dadurch gekennzeichnet, daß man als Chlorierungsmittel IV ein Vilsmeier-Salz oder ein nicht-oxidierendes Chlorierungsmittel verwendet.

3. Verfahren nach Anspruch 2, dadurch gekennzeichnet, daß man als nicht-oxidierendes Chlorierungsmittel IV Sulfurylchlorid, Thionylchlorid, Acetylchlorid, Benzoylchlorid, Pivalinsäurechlorid, Bis-(trichlormethyl)-carbonat, Oxalyldichlorid, Phosphoroxychlorid, Phosphortrichlorid, Phosphorpentachlorid, Methansulfonsäurechlorid, Chlorsulfonsäure, Chlorameisensäuretrichlormethylester oder Phosgen verwendet.

4. Verfahren nach Anspruch 1, dadurch gekennzeichnet, daß man zusätzlich zu IIIa oder IIIb ein inertes Lösungsmittel mit verwendet.

5. Verfahren nach Anspruch 1, dadurch gekennzeichnet, daß man es auf die Umsetzung solcher Verbindungen II anwendet, in denen R¹ einen iso- oder heterocyclischen aromatischen Rest bedeutet.

6. Oxirane der allgemeinen Formel IIa 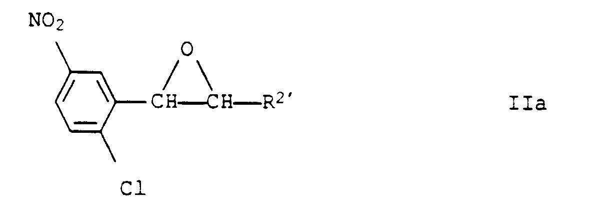 wobei R^{2'} Cyano, C₁-C₆-Alkylcarbonyl, C₁-C₆-Alkoxycarbonyl oder C₁-C₆-Alkylthiocarbonyl bedeuten.

## Claims

1. A process for preparing chlorine-substituted olefinic compounds of the general formula I 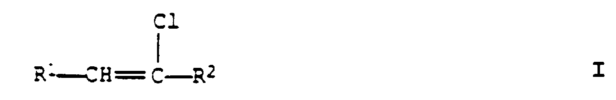 where R¹ is a C-organic radical and R² is one of the following groups: -CN, -CO-R³, -CO-S-R³, -CO-O-R³ or -CO-NR⁴R⁵,
where R³ is a C-organic radical and R⁴ and R⁵ are each hydrogen or a C-organic radical,
which comprises reacting an oxirane of the general formula II 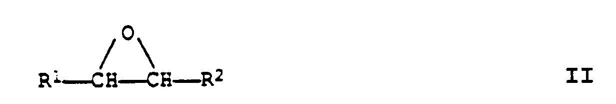 in the presence of a carboxamide (IIIa) or of a lactam (IIIb) in liquid phase with a chlorinating agent (IV).

2. A process as defined in claim 1, wherein a Vilsmeier salt or a non-oxidizing chlorinating agent is used as chlorinating agent IV.

3. A process as defined in claim 2, wherein sulfuryl chloride, thionyl chloride, acetyl chloride, benzoyl chloride, pivaloyl chloride, bis(trichloromethyl) carbonate, oxalyl dichloride, phosphorus oxychloride, phosphorus trichloride, phosphorus pentachloride, methanesulfonyl chloride, chlorosulfonic acid, trichloromethyl chloroformate or phosgene is used as non-oxidizing chlorinating agent IV.

4. A process as defined in claim 1, wherein an inert solvent is used in addition to IIIa or IIIb.

5. A process as defined in claim 1, wherein the proces is used for the reaction of such compounds II in which R¹ is an iso- or heterocyclic aromatic radical.

6. An oxirane of the general formula IIa 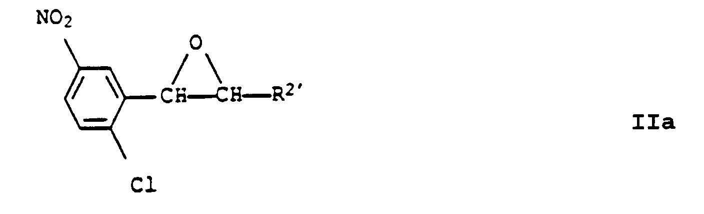 where R^{2'} is cyano, C₁-C₆-alkylcarbonyl, C₁-C₆-alkoxycarbonyl or C₁-C₆-alkylthiocarbonyl.

## Revendications

1. Procédé de préparation de composés oléfiniques substitués par du chlore, de formule générale I 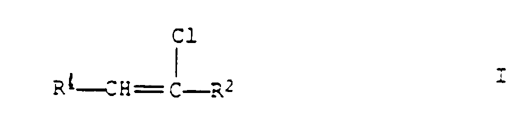 dans laquelle R¹ représente un reste de composé de C et R² un des groupes suivants : -CN, -CO-R³, -CO-S-R³, -CO-R³ ou -CO-N(R⁴,R⁵),
R³ étant mis pour un reste de composé de C et R⁴ et R⁵ pour hydrogène ou reste de composé de C,
caractérisé par le fait que l'on fait réagir un oxiranne de formule générale II 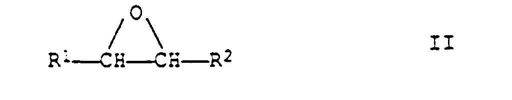 en présence d'un amide d'acide carboxylique (IIIa) ou d'un lactame (IIIb), en phase liquide, avec un agent de chloration (IV).

2. Procédé selon la revendication 1, caractérisé par le fait que l'on utilise, comme agent de chloration IV, un sel de Vilsmeier ou un agent de chloration non oxydant.

3. Procédé selon la revendication 2, caractérisé par le fait que l'on utilise, comme agent de chloration IV non oxydant, du chlorure de sulfuryle, du chlorure de thionyle, de chlorure d'acétyle, de chlorure de benzoyle, de chlorure d'acide pivalinique, du carbonate de bis(trichlorométhyle), du dichlorure d'oxalyle, de l'oxychlorure de phosphore, du trichlorure de phosphore, du pentachlorure de phosphore, du chlorure d'acide méthanesulfonique, de l'acide chlorosulfonique, du chloroformiate de trichlorométhyle ou du phosgène.

4. Procédé selon la revendication 1, caractérisé par le fait que l'on utilise, en plus du IIIa ou IIIb, un solvant inerte.

5. Procédé selon la revendication 1, caractérisé par le fait que l'on utilise sur la réaction des composés II dans lesquels R¹ représente un reste aromatique iso- ou hétérocyclique.

6. Oxiranne de formule générale IIa 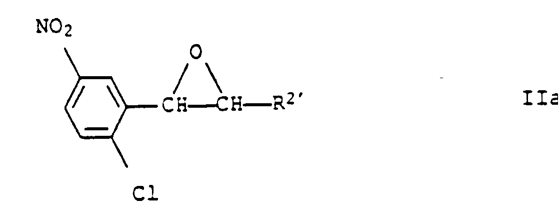 dans laquelle R^{2'} représente cyano, alkylcarbonyle en C₁-C₆, alcoxycarbonyle en C₁-C₆ ou alkylthiocarbonyle en C₁-C₆.
